# EUROPEAN PATENT APPLICATION

(11) **EP 4 813 449 A1**
(43) Date of publication of application: **30.09.2026**
(21) Application number: 25166696.2
(22) Date of filing: 27.03.2025
(51) Int. Cl.: A61N 5/10

(54) **RADIOTHERAPY PATIENT POSITIONING SYSTEM PROTECTIVE COVER**

(71) Applicant: Siemens Healthineers AG, 91301 Forchheim (DE)
(72) Inventor: STOCK, Johannes, 95478 Kemnath (DE); HRUSCHKA, Klaus, 95478 Kemnath (DE); BAUMANN, Berthold, 95478 Kemnath (DE); EIGNER, Daniel, 95478 Kemnath (DE); PLANNERER, Patrick, 95478 Kemnath (DE); HECHT, Jochen Dieter, 91058 Erlangen (DE); SUGAYA, Viola, 5405 Dättwil (CH); KILIAN, Katrin, 95478 Kemnath (DE)
(74) Representative: Mathisen & Macara LLP

(57) **Abstract**

A protective cover 12 for a radiotherapy patient positioning system is disclosed. The radiotherapy patient positioning system has at least two parts that are moveable relative to one another. The protective cover comprises at least a first cover section 16A, 18A, 20A, 22A, 24A, 26A, 27A, that is configured to cover a first of said parts and to overhang a second of the parts of the patient positioning system. The protective cover may include a second cover section that is configured to cover the second of said parts of the patient positioning system, wherein the first cover section is configured to overhang the second cover section. The size and shape of the first cover section and the second cover section are preferably such that liquid F running down the protective cover is guided away from the parts of radiotherapy patient positioning system covered by the first cover section and the second cover section. The first cover section may include at least one lip that is configured to overhang the second of the parts of the patient positioning system.

## Description

### TECHNICAL FIELD

The present invention relates to a protective cover for a radiotherapy patient positioning system that has at least two parts that are moveable relative to one another. The present invention also relates to a radiotherapy patient positioning system covered with the protective cover.

### BACKGROUND

Radiation therapy is a localised treatment for a specific target tissue (a planning target volume), such as a cancerous tumour. Ideally, radiation therapy is performed on the planning target volume that spares the surrounding normal tissue from receiving doses above specified tolerances, thereby minimising risk of damage to healthy tissue. Prior to the delivery of radiation therapy, an imaging system is typically employed to provide a three-dimensional image of the target tissue and surrounding area. From such imaging, the size and mass of the target tissue can be estimated and an appropriate treatment plan generated and planning target volume determined.

So that the prescribed dose is correctly supplied to the planning target volume (i.e., the target tissue) during radiation therapy, the patient should be correctly positioned relative to the linear accelerator that provides the radiation therapy. Typically, dosimetric and geometric data are checked before and during the treatment, to ensure correct patient placement and that the administered radiotherapy treatment matches the previously planned treatment. This process is referred to as image guided radiation therapy (IGRT), and involves the use of an imaging system to view target tissues while radiation treatment is delivered to the planning target volume. IGRT incorporates imaging coordinates from the treatment plan to ensure the patient is properly aligned for treatment in the radiation therapy device.

A radiotherapy patient positioning system is used to support and position the patient during treatment and/or imaging. A radiotherapy patient positioning system typically includes a couch that supports the patient in a supine position. The radiotherapy patient positioning system typically further includes a mechanism for moving the couch (and the supported patient).

The movement between different moveable parts of a radiotherapy patient positioning system result in a device that has a changeable size and shape. The movement between different moveable parts of a radiotherapy patient positioning system can make it difficult to keep the radiotherapy patient positioning system clean and to prevent the ingress of fluids (for example, from the patient).

A radiotherapy patient positioning system may have six axes of movement. Providing the six axes is a particular challenge, as the complex mobility of the axes is characterised by the high number of degrees of freedom. To ensure the functionality of the radiotherapy patient positioning system, the degrees of freedom must be strictly adhered to in order to avoid collisions between the static and moving parts. This creates an increased risk of pinch points, especially at the transitions between the axles and other moving components, which poses both safety concerns and technical challenges.

Another problem is the cleanability of the components. The large number of movements and axes makes it difficult to access certain areas, which makes thorough cleaning complicated. This can be a serious problem in environments where cleanliness plays a crucial role, such as therapy systems.

In addition, there is a risk that hoses, cables or other accessories that move with the axes could become trapped or damaged during operation. This requires special precautions when guiding lines and selecting accessories to ensure that they can follow the movements of the axes without being blocked or damaged. Appropriate gap dimensions must also be observed in accordance with the IEC.

It has been proposed to cover a vertical section of a radiotherapy patient positioning system, which provides heigh adjustment, with bellows - which help to protect mechanical components by providing flexible covers. These bellows are often made of elastomers, which are known for their flexibility and elasticity. However, elastomers can become brittle over time, especially when exposed to extreme temperatures, UV radiation or chemical stresses. Brittle bellows lose their protective function and can easily crack or break, leaving the underlying components unprotected. This increases the risk of contamination, mechanical wear or damage caused by external influences. In addition, the production of bellows is complex and must be secured against pressure accordingly so that there is no unintentional penetration into moving mechanics.

### SUMMARY

In one aspect, the present invention provides a protective cover for a radiotherapy patient positioning system having at least two parts that are moveable relative to one another, the protective cover comprising at least a first cover section that is configured to cover a first of said parts and to overhang a second of said parts.

By the first cover section being configured to overhang the second of said parts, the first cover section may divert liquid away from the second of said parts to reduce the likelihood that liquid flowing down the first cover section will enter the second of said parts and potentially cause damage.

The protective cover may include a second cover section that is configured to cover the second of said parts, wherein the first cover section is configured to overhang the second cover section. The second cover section may protect the second of said parts. By the first cover section being configured to overhang the second cover section, the first cover section may divert liquid away from any gap between the first and second cover sections to reduce the likelihood that liquid flowing down the first cover section will enter the second of said parts and potentially cause damage.

The size and shape of the first cover section and the second cover section may be such that liquid running down the protective cover is guided away from the parts of radiotherapy patient positioning system covered by the first cover section and the second cover section.

The first cover section may be configured to partially overlap the second cover section. By the first cover section being configured to partially overlap the second cover section this may reduce the likelihood that liquid flowing down the first cover section will enter the second of said parts and potentially cause damage and may reduce the likelihood of any components getting caught between the first and second cover sections

The first cover section may be slidably coupled to the second cover section to accommodate movement between the first of said parts and the second of said parts of the radiotherapy patient positioning system.

The first cover section and the second cover section may comprise a telescopic side wall structure, wherein the telescopic side wall structure is shaped to fit around the two parts of the positioning system that are moveable relative to one another and the telescopic side wall structure is configured to expand or contract with relative movement of the two parts of the positioning system. Preferably, the first cover section may have a first side wall structure that overlaps a second side wall structure of the second cover section by a variable amount to accommodate relative movement of the two parts of the positioning system, wherein a lower edge of the first side wall structure includes an inwardly extending flange and an upper edge of the second side wall structure includes a outwardly extending flange, the inwardly extending flange and the outwardly extending flange being configured to engage one another to prevent the first side wall structure and the second side wall structure separating in response to the relative movement of the two parts of the positioning system. Such first and second cover sections may form a vertical support cover section in the embodiment to be described.

The first cover section may include at least one lip that is configured to overhang the second of said parts. The at least one lip may be configured to partially overlap the second of said parts. The at least one lip may extend in a first direction along a side of the first cover section and may be configured to allow movement of the first of said parts relative to the second of said parts in the first direction. Such a first cover section may form a couch cover section in the embodiment to be described. The second of the parts may be covered by a second cover section.

The first cover section may extend generally vertically, the protective cover comprising a further cover section that extends generally horizontally, the further cover section being provided with one or more drainage holes. Such a further cover section may form part of a longitudinal coupling cover section in the embodiment to be described.

The first cover section may extend generally vertically, the protective cover comprising a further cover section that extends generally horizontally, the further cover section being provided with one or more protrusions for guiding flow of liquid in a desired direction. Such a further cover section may form part of a foot cover section in the embodiment to be described.

The sections of the cover may, for example, be one of, or a part of, any of the following:
- A footrest cover section that is configured to cover a footrest of the radiotherapy patient positioning system.
- A pitch roll cover section that is configured to cover a pitch roll coupling of the radiotherapy patient positioning system.
- A longitudinal coupling cover section that is configured to cover a longitudinal coupling of the radiotherapy patient positioning system.
- A vertical support cover section that is configured to cover a vertical support of the radiotherapy patient positioning system.
- One of several cover parts comprising the vertical support cover section.
- A foot cover section that is configured to cover a foot of the radiotherapy patient positioning system.
- A base cover section that is configured to cover a base of the radiotherapy patient positioning system.
- A yaw module cover section that is configured to cover a yaw module of the radiotherapy patient positioning system.

In another aspect, the present invention provides a radiotherapy patient positioning system covered with the protective cover as specified above.

A radiotherapy patient positioning apparatus according to a further aspect of the invention includes:
a radiotherapy patient positioning system having at least two parts that are moveable relative to one another; and
a protective cover comprising at least a first cover section arranged to cover a first of said parts and to overhang a second of said parts.

A second cover section may be arranged to cover the second of said parts, wherein the first cover section overhangs the second cover section.

The size and shape of the first cover section and the second cover section may be such that liquid running down the protective cover is guided away from the parts of radiotherapy patient positioning system covered by the first cover section and the second cover section.

The first cover section may partially overlap the second cover section.

The first cover section may be slidably coupled to the second cover section to accommodate movement between the first of said parts and the second of said parts of the radiotherapy patient positioning system.

The first cover section and the second cover section may comprise a telescopic side wall structure, wherein the telescopic side wall structure is shaped to fit around the two parts of the positioning system that are moveable relative to one another and the telescopic side wall structure is configured to expand or contract with relative movement of the two parts of the positioning system.

The first cover section may have a first side wall structure that overlaps a second side wall structure of the second cover section by a variable amount to accommodate relative movement of the two parts of the positioning system, wherein a lower edge of the first side wall structure includes an inwardly extending flange and an upper edge of the second side wall structure includes a outwardly extending flange, the inwardly extending flange and the outwardly extending flange being configured to engage one another to prevent the first side wall structure and the second side wall structure separating in response to the relative movement of the two parts of the positioning system.

The first cover section may include at least one lip that overhangs the second of said parts.

The at least one lip may partially overlap the second of said parts.

The at least one lip may extend in a first direction along a side of the first cover section and may be configured to allow movement of the first of said parts relative to the second of said parts in the first direction.

The size and shape of the first cover section may be such that liquid running down the protective cover is guided away from the second of said parts of radiotherapy patient positioning system.

The first cover section may extend generally vertically, the protective cover comprising a further cover section that extends generally horizontally, the further cover section being provided with one or more drainage holes.

The first cover section may extend generally vertically, the protective cover comprising a further cover section that extends generally horizontally, the further cover section being provided with one or more protrusions for guiding flow of liquid in a desired direction.

The overlapping cover design from top to bottom of the cover may be based on the so-called roof tile principle, where the covers or protective layers are arranged in such a way that they overlap and form a seamless barrier. Similar to roof tiles, each top cover is placed over the one below to prevent liquids, dust, or other foreign objects from entering. This may create an effective protective layer that is particularly useful in applications where sensitive or moving parts need to be protected from external influences.

The nesting of the covers takes place from the inside to the outside, so that no (or little) liquid or contamination may get to the load-bearing or moving, driven parts of the radiotherapy treatment system. This arrangement may be particularly beneficial in applications where liquids such as urine, water or detergents are used, as it provides that they tend not to penetrate into areas where they could cause damage or malfunction. The overlapping and nesting keeps the mechanics underneath protected, increasing the longevity and reliability of the entire system.

In summary, this design may offer robust protection against external influences due to its overlapping and nesting and is ideal for applications where moving and driven parts are particularly sensitive to liquids and dirt.

Embodiments may provide:
- Simplified assembly due to reduced component scope, giving a cost advantage
- Integrated interfaces on a highly integrated component in the assembly for e.g. exact movement of the patient along an axis
- Complex geometries in the product can be implemented

### BRIEF DESCRIPTION OF DRAWINGS

The accompanying drawings, which are incorporated in and form a part of this specification and in which like numerals depict like elements, illustrate embodiments of the present disclosure and, together with the detailed description, serve to explain the principles of the disclosure.
Figure 1 is a schematic perspective view of a radiation treatment system.
Figure 2 is front elevation of a radiotherapy patient positioning system indicating the position of parts of the system.
Figure 3 is a perspective front and right side view of the radiotherapy patient positioning system including a cover (left side corresponds).
Figure 4 is front elevation of the radiotherapy patient positioning system including the cover.
Figure 5 is a perspective rear and right side view of the radiotherapy patient positioning system including the cover.
Figure 6 is an enlarged partial front elevation of the radiotherapy patient positioning system including the cover, showing a footrest cover section, a pitch roll cover section and a longitudinal coupling cover section.
Figure 7 is an enlarged partial vertical cross-section of the radiotherapy patient positioning system including the cover, showing part of the footrest cover section, the pitch roll cover section and part of the longitudinal coupling cover section.
Figure 8 is an enlarged partial vertical cross-section of the radiotherapy patient positioning system including the cover, showing part of the pitch roll cover section and the longitudinal coupling cover section.
Figure 9 is an enlarged partial vertical cross-section of the radiotherapy patient positioning system including the cover, showing part of the longitudinal coupling cover section, a vertical support cover section and part of a foot cover section.
Figure 10 is an enlarged partial vertical cross-section of the radiotherapy patient positioning system including the cover, showing part of the vertical support cover section, the foot cover section and part of a base cover section.
Figure 11 is an enlarged partial perspective front and left side view of part of the vertical support cover section, the foot cover section and the base cover section.

### DETAILED DESCRIPTION

Reference will now be made in detail to the various embodiments of the present disclosure, examples of which are illustrated in the accompanying drawings. While described in conjunction with these embodiments, it will be understood that they are not intended to limit the disclosure to these embodiments. On the contrary, the disclosure is intended to cover alternatives, modifications and equivalents, which may be included within the scope of the disclosure as defined by the appended claims. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

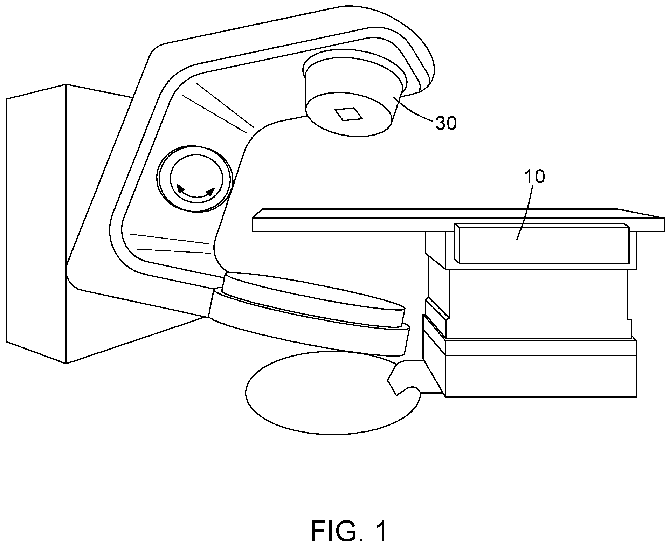

Figure 1 depicts a radiation treatment system. Typically, such a system is capable of generating either an electron (particle) beam or an x-ray (photon) beam for use in the radiotherapy treatment of patients on a radiotherapy patient positioning system 10. Other radiation treatment systems are capable of generating heavy ion particles such as protons. For purposes of the present discussion, only x-ray irradiation will be discussed. However, it will be appreciated by those skilled in the art that the same principles apply to other systems.

Stand 1 supports a rotatable gantry 2 with a treatment head 3. Next to stand 1 there is arranged a control unit (not shown) that includes control circuitry for controlling the different modes of operation of the accelerator. A high voltage source is provided within the stand or in the gantry, to supply voltage to an electron gun (not shown) positioned on an accelerator guide located in the gantry 2. Electrons are emitted from the electron gun into the guide (not shown) where they are accelerated. A source supplies RF (microwave) power for the generation of an electric field within the waveguide. The electrons emitted from the electron gun are accelerated in the waveguide by the electric field, and exit the waveguide as a high energy electron beam, typically at megavoltage energies. The electron beam then strikes a suitable metal target, emitting high energy x-rays in the forward direction.

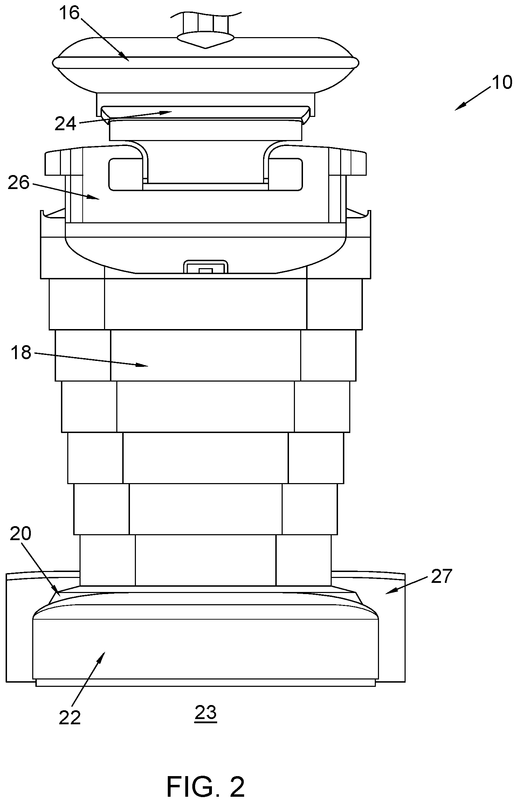

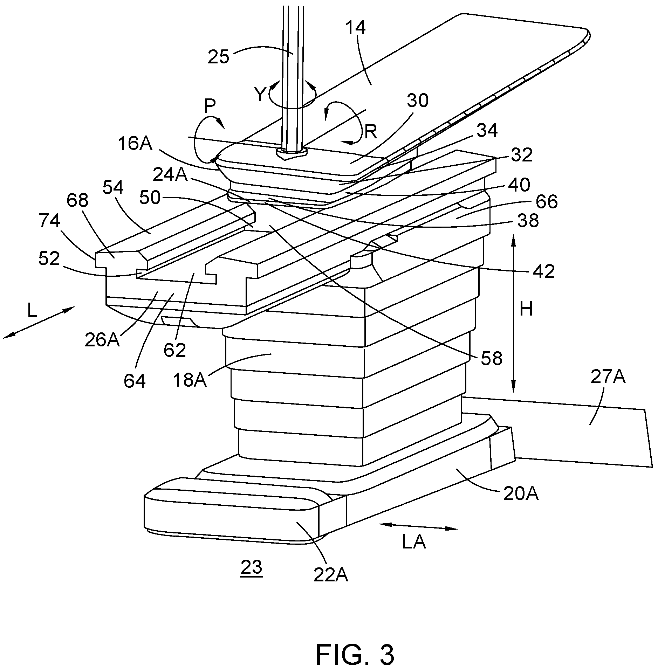

Figure 2 is front elevation of a radiotherapy patient positioning system 10. Figure 2 is intended to show the approximate location of parts of the patient positioning system 10 but does not show these parts individually in detail. The radiotherapy patient positioning system 10 includes a patent couch 14 (see Figure 3) having a footrest at location 16, a vertical support at location 18 having a foot at location 20, and a base at location 22 that is in contact with a floor 23. A camera post 25 may be fitted to the patient couch 14. The patient couch 14 may be made from carbon fibre.

The couch 14 is coupled at the footrest end to the vertical support by a pitch roll coupling at location 24 and a longitudinal coupling at location 26. The footrest end of the vertical support is connected to the pitch roll coupling. The pitch roll coupling is connected to the vertical support by the longitudinal coupling. The longitudinal coupling is connected to the vertical support. The foot of the vertical support is connected to the base.

The pitch roll coupling allows movement of the patent couch 14 with respect to the base about a roll axis "R" (see Figure 3). The pitch roll coupling also allows movement of the patent couch 14 with respect to the base about a pitch axis "P". The longitudinal coupling allows movement of the patent couch 14 with respect to the base along a longitudinal axis "L". The pitch roll coupling is configured to slide along the longitudinal coupling in the direction of the longitudinal axis "L". The vertical support allows the height of the patent couch 14 to vary with respect to the base along a vertical axis "H" that is perpendicular to the longitudinal axis "L".

The foot of the vertical support is coupled to the base such that the foot can move with respect to the base in a lateral direction "LA" that is perpendicular to the vertical axis "H" and that is perpendicular to the longitudinal axis "L". The foot 20 of the vertical support 18 is coupled to the base 22 such that the foot 20 can move with respect to the base 22 about a yaw axis "Y". Movement about the yaw axis "Y" is provided by a yaw module at location 27. Due to the (indirect) coupling of the patient couch 14 to the foot, the movement of the foot of the vertical support with respect to the base allows the patient couch 14 to move in the lateral direction "LA" and about the yaw axis "Y".

The connections described above provide the patient couch 14 with six movement axes, and so allow a patient to be positioned precisely and accurately. The connections and movements described above are generally known and may be implemented by any suitable arrangement - and are not described in detail herein. Fewer than, or more than, six axes of movement may be provided.

The terms "vertical" and "vertically", and "horizontal" and "horizontally" used herein should not be taken to require a particular orientation of any part when deployed, but are used to describe relative orientations of parts as shown in the figures.

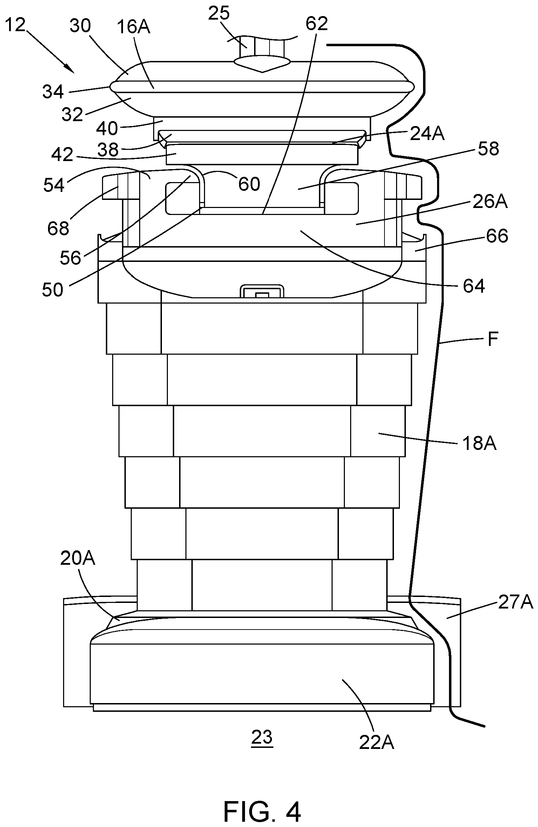

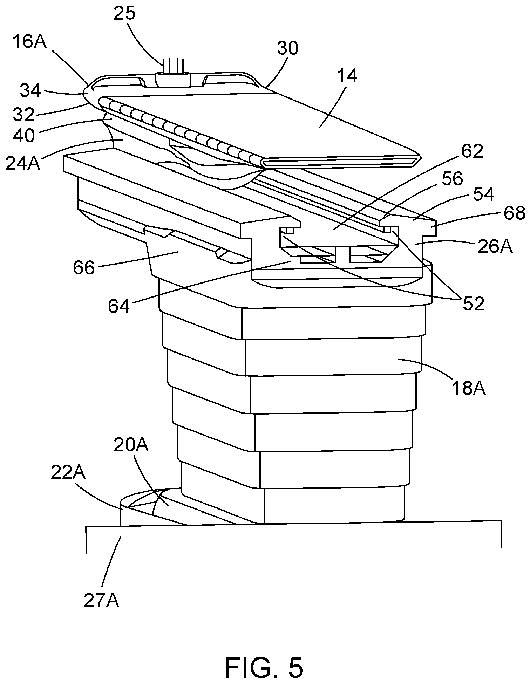

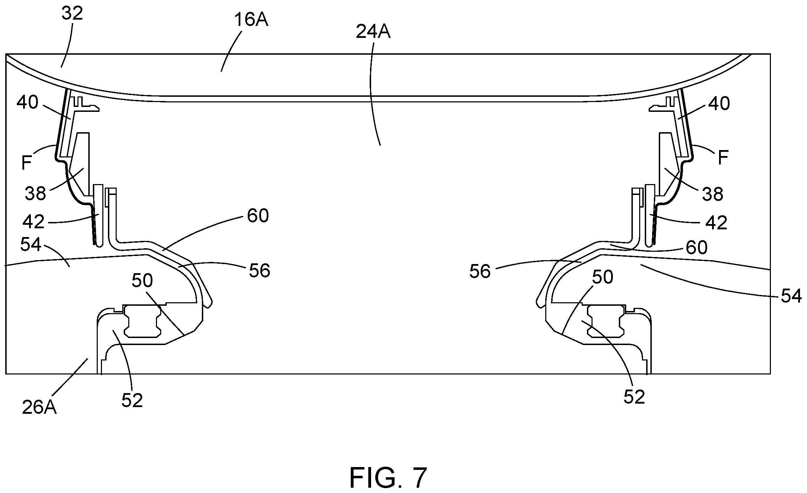

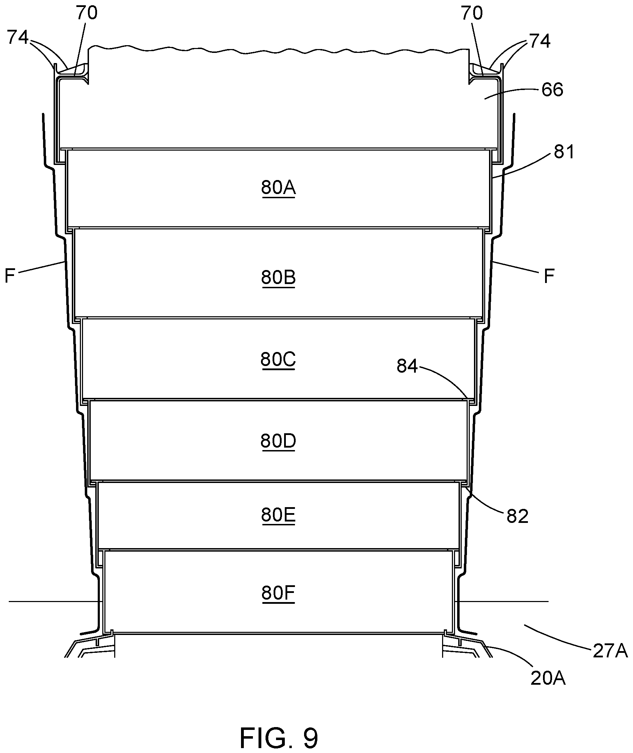

The radiotherapy patient positioning system 10 is provided with a cover 12 in accordance with an embodiment of the invention, as will be described with reference to Figures 3 to 11. The cover 12 comprises a plurality of sections:
- A footrest cover section 16A that is configured to cover the foot rest 16.
- A pitch roll cover section 24A that is configured to cover the pitch roll coupling 24. The pitch roll cover section 24A is located below the footrest cover section 16A.
- A longitudinal coupling cover section 26A that is configured to cover the longitudinal coupling 26. The longitudinal coupling cover section 26A is located generally below the pitch roll cover section 24A.
- A vertical support cover section 18A that is configured to cover the vertical support 18. The vertical support cover section 18A is located below the longitudinal coupling cover section 26A.
- A foot cover section 20A that is configured to cover the foot 20. The foot cover section 20A is located below the vertical support cover section 18A.
- A base cover section 22A that is configured to cover the base 22.
- A yaw module cover section 27A that is configured to cover the yaw module 27.

The parts of the patient positioning system (e.g. the footrest 16, the pitch roll coupling 24, longitudinal coupling 26, vertical support 18, foot 20, base 22 and/or yaw module 27) are covered by one of more of the sections of the cover 12. The parts of the patient positioning system are moveable relative to one another to provide the desired movement of the patient positioning system to support a patient on the couch 14 in a desired position.

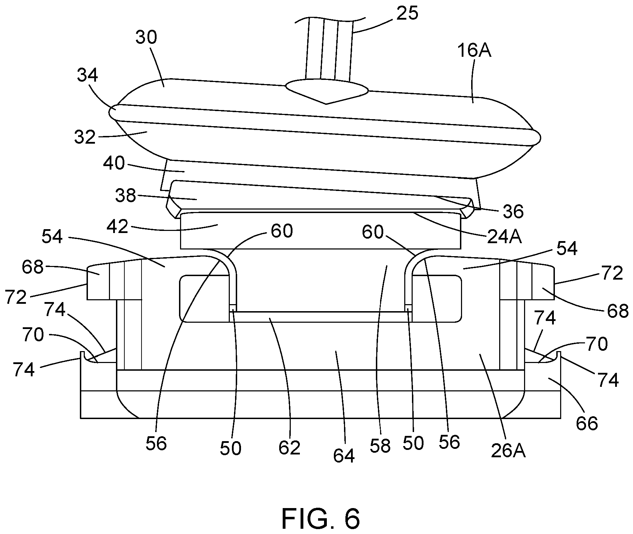

The footrest cover section 16A encases one end of the patient couch 14, at the footrest 16. As best shown in Figure 6, the footrest cover section 16A includes an upper footrest cover section portion 30 that covers the upper main surface of the footrest 16, a lower footrest cover section portion 32 that partially covers the lower main surface of the footrest 16 and a peripheral footrest cover section portion 34 that extends around the periphery of the footrest 16 that connects the upper footrest cover section portion 30 and the lower footrest cover section portion 32. The lower footrest cover section portion 32 tapers downwardly towards a generally rectangular footrest cover section lower opening 36 in the lower footrest cover section portion 32. The generally rectangular lower footrest cover section opening 36 is sized and located to fit over an upper generally rectangular (in horizontal cross-section) pitch roll cover section portion 38 of the pitch roll cover section 24A. The lower footrest cover section portion 32 includes a lower footrest cover section portion lip 40 that extends downwardly around the lower footrest cover section opening 36, overhanging and partially overlapping the exterior of the upper generally rectangular pitch roll cover section portion 38 on three or more sides. The upper footrest cover section portion 30, lower footrest cover section portion 32, peripheral footrest cover section portion 34 and lower footrest cover section portion lip 40 may be integrally formed.

The lower footrest cover section portion lip 40 and the upper generally rectangular pitch roll cover section portion 38 are sized and shaped such that the lower footrest cover section portion lip 40 can slide over and tilt with respect to the lower footrest cover section portion 38 to accommodate movement of the patient couch 14 about the roll axis "R" and the pitch axis "P". For example, Figure 6 shows a roll angle of 3°. The lower footrest cover section portion lip 40 always at least partially overlaps the upper generally rectangular pitch roll cover section portion 38, even at the extremes of permitted movement about the roll axis "R" and the pitch axis "P" - and this prevents, or reduces, dirt or fluid entering the pitch roll coupling at location 24 and/or hoses, cables or other accessories being trapped between the patient couch 14 and the pitch roll coupling at location 24.

The upper generally rectangular pitch roll cover portion 38 of the pitch roll cover section 24A extends around and partially overlaps the exterior of a generally vertically extending middle pitch roll cover portion 42 of the pitch roll cover section 24A - and this prevents, or reduces, dirt or fluid entering the pitch roll coupling located at 24.

As mentioned above, the pitch roll coupling located at 24 is connected to the vertical support located at 18 by the longitudinal coupling located at 26. The pitch roll coupling includes a flange 50 (best seen in Figure 3) that slides within channels 52 at opposite sides of the longitudinal coupling located at 26, and this allows movement of the pitch roll coupling (and the attached patient couch 14) along the longitudinal direction "L".

An upper part of each channel 52 is formed by a generally horizontal wall 54 of the longitudinal coupling cover section 26A. The generally horizontal walls 54 of the longitudinal coupling cover section 26A each have a curved distal end 56. A lower pitch roll cover portion 58 of the pitch roll cover section 24A extends from the lower end of the middle pitch roll cover portion 42 of the pitch roll cover section 24A to the flange 50. The lower pitch roll cover portion 58 curves inwardly at curved portions 60 to clear the generally horizontal walls 54 and curved distal ends 56 of the longitudinal coupling cover section 26A and then extends generally vertically towards the flange 50.

Liquid running down the generally vertically extending middle pitch roll cover portion 42 of the pitch roll cover section 24A will drip onto the generally horizontal walls 54 of the longitudinal coupling cover section 26A and may flow outwardly, away from the channels 52 or inwardly towards the channels 52. The end of the vertically extending middle pitch roll cover portion 42, where it meets or lies adjacent to the lower pitch roll cover portion 58, may form a "dripping edge".

Liquid running down the generally vertically extending middle pitch roll cover portion 42 of the pitch roll cover section 24A that flows inwardly towards the channels 52 may be collected in a bucket or reservoir associated with the flange 50. Liquid running down the generally vertically extending middle pitch roll cover portion 42 of the pitch roll cover section 24A that flows inwardly towards the channels 50 may alternatively, or additionally, reach a main horizontal surface 62 of a main body 64 of the longitudinal coupling cover section 26A, the main horizontal surface 62 lying between the channels 52. The main horizontal surface 62 of the longitudinal coupling cover section 26A may be provided with one or more drain holes to allow the liquid to be drained away from the radiotherapy patient positioning system 10. The main horizontal surface 62 of the longitudinal coupling cover section 26A may be formed of metal.

The longitudinal coupling cover section 26A includes a lower engagement portion 66 that is coupled to the vertical cover section located at 18. The lower engagement portion 66 extends around and partially overlaps the vertical cover section 18 - and this prevents, or reduces, dirt or fluid entering the vertical cover section located at 18.

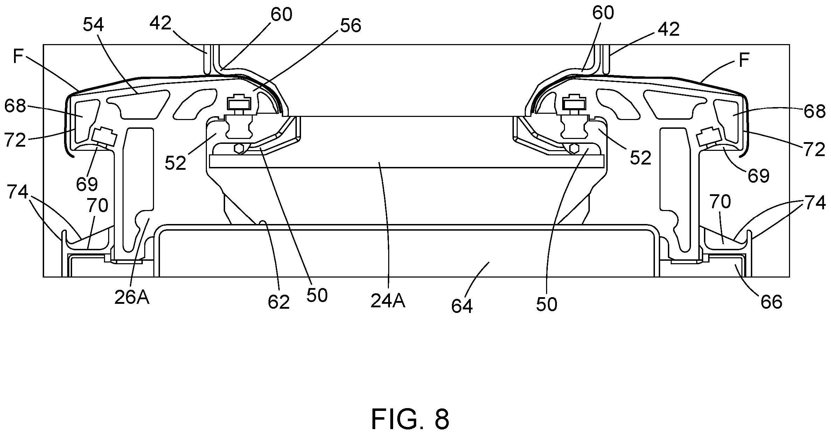

The main body 64 of the longitudinal coupling cover section 26A is fixed to the lower engagement portion 66. The generally horizontal wall 54 of the longitudinal coupling cover section 26A extends outwardly beyond the main body 64 to form protrusions 68. The downwardly facing surface 69 of the protrusions 68 may be concave (see Figure 8).

An upper peripheral surface 70 of the lower engagement portion 66 extends generally horizontally outwardly beyond the generally horizontal wall 54 (in the lateral direction "LA"). Liquid running down the generally vertically extending middle pitch roll cover portion 42 of the pitch roll cover section 24A that drips onto the generally horizontal walls 54 of the longitudinal coupling cover section 26A and flows outwardly, away from the channels 52 will then flow down a vertical surface 72 of the protrusions 68. The protrusions overhang the main body 64. Fluid flowing down the vertical surface 72, when reaching the corner with the downwardly facing surface 69 then drips onto the upper peripheral surface 70 of the engagement portion 66. The concave shape of the downwardly facing surface 69 of the protrusions 68 prevents or reduces any flow of liquid along the downwardly facing surface 69. The corner of the vertical surface 72 with the downwardly facing surface 69 may form a "dripping edge". Fluid collection walls 74 may extend upwardly from the outer edges of the upper peripheral surface 70 of the engagement portion 66 to form a bucket or reservoir to collect the liquid.

The vertical cover section 18 includes a plurality (six in this example) of similar, but not identical, vertical cover parts 80A to 80F. Each of the vertical cover parts 80A to 80F is a rectangular hollow section part. That is, each of the vertical cover parts 80A to 80F comprises a four-sided generally vertical wall 81 providing a hollow cuboid space within. The lower engagement portion 66 of the longitudinal coupling cover section cover 26A is coupled to the uppermost vertical cover part 80A of the vertical cover section 18. The lower engagement portion 66 extends around, overhangs and partially overlaps the uppermost vertical cover part 80A. Each of the vertical cover parts 80B to 80F is smaller in width (in along the axis "LA") and is smaller in depth (along the axis "L") than the vertical cover part immediately above, so that a lower one of the vertical cover parts 80B to 80F can slide within (along axis "H) the vertical cover parts 80A to 80E immediately above. In this way, the vertical cover parts 80A to 80F comprise a telescopic side wall structure. The telescopic side wall structure is shaped to fit around parts of the positioning system that are moveable relative to one another and the telescopic side wall structure is configured to expand or contract with relative movement of the parts of the positioning system. Such an arrangement allows the height H of the vertical cover section 18 to vary, and therefore the height of the patient couch 14 to vary.

The side wall structure of each vertical cover part 80A to 80F overlaps the side wall structure of an adjacent vertical cover part 80A to 80F. The amount of overlap is variable to accommodate relative movement of the parts of the positioning system.

Referring, for example, to vertical cover part 80D, a lower edge of its side wall structure includes an inwardly extending flange 82 and an upper edge of its side wall structure includes an outwardly extending flange 84. The other vertical cover parts 80A-C and 80E-F include similar inwardly extending flanges 82 and outwardly extending flanges 84 at the lower and upper edges of their side wall structures, respectively. The inwardly extending flange 84 of vertical cover part 80C and the outwardly extending flange 84 of vertical cover part 80D are configured to engage one another to prevent the side wall structure of vertical cover part 80C and the side wall structure of vertical cover part 80D separating in response to the relative movement of the two parts of the positioning system. The inwardly extending flange 82 and the outwardly extending flange 84 of each adjacent vertical cover part are similarly configured to engage one another to prevent the side wall structures of adjacent vertical cover parts separating. Each vertical cover part always at least partially overlaps the adjacent lower vertical cover part, even at the extremes of permitted movement - and this prevents, or reduces, dirt or fluid entering the vertical cover section 18 and/or hoses, cables or other accessories being trapped between the vertical cover parts 80A to 80F.

The foot cover 20A is fixed to the lowermost vertical cover part 80F of the vertical support located at 18. As mentioned above, the foot located at 20 of the vertical support located at 18 is coupled to the base located at 22 such that the foot can move with respect to the base in a lateral direction "LA" that is perpendicular to the vertical axis "H" and that is perpendicular to the longitudinal axis "L". The foot of the vertical support is coupled to the base such that the foot can rotate with respect to the base about a yaw axis "Y". Movement about the yaw axis "Y" is provided by a yaw module located at 27. Due to the (indirect) coupling of the patient couch 14 to the foot, the movement of the foot of the vertical support with respect to the base allows the patient couch 14 to move in the lateral direction "LA" and about the yaw axis "Y".

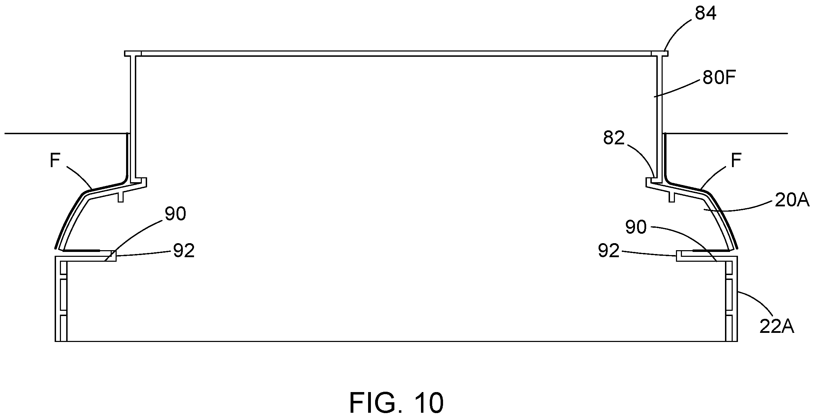

As shown in Figure 10, an upper generally horizontal surface 90 of the base cover 22A extends under the lower distal edge of the foot cover 20A. Upper generally horizontal surface 90 may include an upstanding wall 92 at its innermost edge. Fluid flowing down the foot cover 20A will generally then flow down the vertical surface of the base cover 22A. A small portion of the fluid flowing down the foot cover 20A may then flow along upper generally horizontal surface 90 but the upstanding wall 92 and the small inclination of the upper generally horizontal surface 90 will cause any such fluid to be redirected down the vertical surface of the base cover 22A. The distal edge of the foot cover 20A may form a "dripping edge".

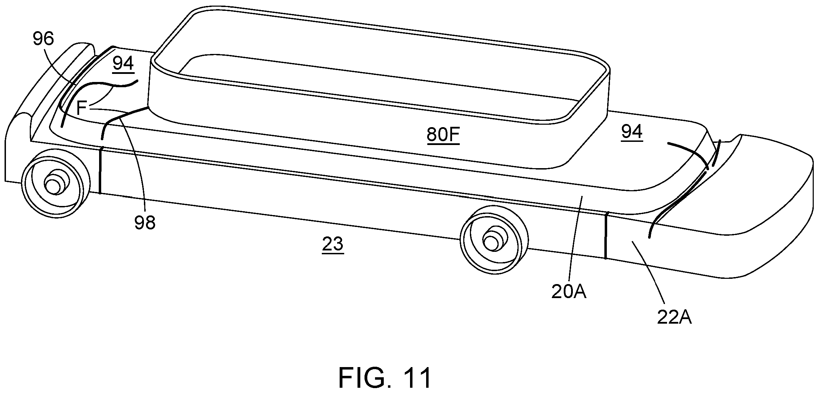

As shown in Figure 11, the foot cover 20A may have a generally horizontal surface 94 that is attached to the lowermost vertical cover part 80F of the vertical support located at 18. The generally horizontal surface 94 may include one or more fluid-guiding walls or protrusions 96 to direct the flow of fluid along a desired path. As also shown in Figure 11, the generally horizontal surface 94 may include one or more channels 98 to direct the flow of fluid along a desired path - e.g. to a particular part of the base cover section 22A. The channels 98 may be formed by tongue and groove connections between parts forming the horizontal surface 94.

The cover 12 prevents, or reduces, dirt or fluid entering the radiotherapy patient positioning system 10 that is inside the cover 12 and also prevents hoses, cables or other accessories being trapped between parts of the radiotherapy patient positioning system 10 that is inside the cover 12. The general path of fluid along the cover 12 is indicated in the figures by letter "F".

The parts of the cover 12 may be made from plastic or any other suitable material. A robust and rigid material is preferred.

The parts of the cover 12 may have seals provided between them.

The parts of the cover 12 are easy to clean, which provides a hygienic design for the operator and patient.

The parts of the cover 12 do not restrict the usual movement of the radiotherapy patient positioning system 10. For example, the six degrees of movement are not inhibited by the parts of the cover 12.

The parts of the cover 12 may be compatible with the current existing mechanics and systems which are already installed, and may be retro-fitted to already deployed radiotherapy patient positioning systems.

Although an embodiment has been described in which the cover 12 comprises a plurality of sections, it should be understood that each section alone is a cover for a radiotherapy patient positioning system that may cover only a portion of the radiotherapy patient positioning system. Thus, a cover for a radiotherapy patient positioning system may include any one of:
- A footrest cover section 16A that is configured to cover the foot rest 16.
- A pitch roll cover section 24A that is configured to cover the pitch roll coupling 24.
- A longitudinal coupling cover section 26A that is configured to cover the longitudinal coupling 26.
- A vertical support cover section 18A that is configured to cover the vertical support 18.
- A foot cover section 20A that is configured to cover the foot 20.
- A base cover section 22A that is configured to cover the base 22.
- A yaw module cover section 27A that is configured to cover the yaw module 27.
A cover for a radiotherapy patient positioning system may include any combination of these sections. The vertical support cover section 18A may be particularly suitable for stand-alone use with a radiotherapy patient positioning system, including retro-fitting to an already deployed radiotherapy patient positioning system.

## Claims

1. A protective cover for a radiotherapy patient positioning system having at least two parts that are moveable relative to one another, the protective cover comprising at least a first cover section that is configured to cover a first of said parts of the radiotherapy patient positioning system and to overhang a second of said parts of the radiotherapy patient positioning system.

2. The protective cover of claim 1, including a second cover section that is configured to cover the second of said parts, wherein the first cover section is configured to overhang the second cover section.

3. The protective cover of claim 2, wherein size and shape of the first cover section and the second cover section are such that liquid running down the protective cover is guided away from the parts of radiotherapy patient positioning system covered by the first cover section and the second cover section.

4. The protective cover of claim 2 or 3, wherein the first cover section is configured to partially overlap the second cover section.

5. The protective cover of claim 4, wherein the first cover section is slidably coupled to the second cover section to accommodate movement between the first of said parts and the second of said parts of the radiotherapy patient positioning system.

6. The protective cover of any one of claims 2 to 5, wherein the first cover section and the second cover section comprise a telescopic side wall structure, wherein the telescopic side wall structure is shaped to fit around the two parts of the positioning system that are moveable relative to one another and the telescopic side wall structure is configured to expand or contract with relative movement of the two parts of the positioning system.

7. The protective cover of claim 6, wherein the first cover section has a first side wall structure that overlaps a second side wall structure of the second cover section by a variable amount to accommodate relative movement of the two parts of the positioning system, wherein a lower edge of the first side wall structure includes an inwardly extending flange and an upper edge of the second side wall structure includes a outwardly extending flange, the inwardly extending flange and the outwardly extending flange being configured to engage one another to prevent the first side wall structure and the second side wall structure separating in response to the relative movement of the two parts of the positioning system.

8. The protective cover of claim 1, wherein the first cover section includes at least one lip that is configured to overhang the second of said parts.

9. The protective cover of claim 7, wherein the at least one lip is configured to partially overlap the second of said parts.

10. The protective cover of claim 7 or 8, wherein the at least one lip that extends in a first direction along a side of the first cover section and is configured to allow movement of the first of said parts relative to the second of said parts in the first direction.

11. The protective cover of claim 8, 9 or 10, wherein size and shape of the first cover section is such that liquid running down the protective cover is guided away from the second of said parts of radiotherapy patient positioning system.

12. The protective cover of any one of claims 1 to 11, wherein the first cover section extends generally vertically, the protective cover comprising a further cover section that extends generally horizontally, the further cover section being provided with one or more drainage holes.

13. The protective cover of any one of claims 1 to 12, wherein the first cover section extends generally vertically, the protective cover comprising a further cover section that extends generally horizontally, the further cover section being provided with one or more protrusions for guiding flow of liquid in a desired direction.

14. A radiotherapy patient positioning system covered with the protective cover as defined in any of claims 1 to 13.

15. A radiotherapy patient positioning apparatus including:
a radiotherapy patient positioning system having at least two parts that are moveable relative to one another; and
a protective cover as claimed in any one of claims 1 to 13.
